(19) Europäisches Patentamt
European Patent Office
Office européen des brevets

(11) **EP 2 662 111 B1**

(12) **FASCICULE DE BREVET EUROPEEN**

(45) Date de publication et mention de la délivrance du brevet:
**06.09.2017 Bulletin 2017/36**

(51) Int Cl.:
***A61N 1/36*** *(2006.01)*

(21) Numéro de dépôt: **13166536.6**

(22) Date de dépôt: **03.05.2013**

(54) **Dispositifs de stimulation électrique d'un tissu biologique**

Vorrichtung zur elektrischen Stimulation von biologischem Gewebe

Devices for electrical stimulation of a biological tissue

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.05.2012 FR 1254166**

(43) Date de publication de la demande:
**13.11.2013 Bulletin 2013/46**

(73) Titulaire: **COMMISSARIAT À L'ÉNERGIE ATOMIQUE ET AUX ÉNERGIES ALTERNATIVES 75015 Paris (FR)**

(72) Inventeurs:
• **Dupont, Florent**
  **92320 CHATILLON (FR)**
• **Beche, Jean-François**
  **69100 VILLEURBANNE (FR)**
• **Condemine, Cyril**
  **38140 IZEAUX (FR)**
• **Pham, Pascale**
  **38920 CROLLES (FR)**

(74) Mandataire: **Gevers & Orès**
  **41 avenue de Friedland**
  **75008 Paris (FR)**

(56) Documents cités:
**WO-A2-2006/044793    US-B1- 7 974 696**

• **IONESCU C ET AL: "Activating Paralyzed Muscles using Adaptive Control and a Neuroprosthetic Technique", AUTOMATION, QUALITY AND TESTING, ROBOTICS, 2006 IEEE INTERNATIONAL CON FERENCE ON, IEEE, PI, 1 mai 2006 (2006-05-01), pages 43-47, XP031023907, DOI: 10.1109/AQTR.2006.254494 ISBN: 978-1-4244-0360-8**
• **HAN-CHANG WU ET AL: "A Versatile Multichannel Direct-Synthesized Electrical Stimulator for FES Applications", IEEE TRANSACTIONS ON INSTRUMENTATION AND MEASUREMENT, IEEE SERVICE CENTER, PISCATAWAY, NJ, US, vol. 51, no. 1, 1 février 2002 (2002-02-01), XP011025494, ISSN: 0018-9456**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention se rapporte au domaine technique de la stimulation électrique de tissus biologiques, et notamment de la neurostimulation. A cet effet, l'invention propose un stimulateur électrique comprenant des moyens permettant son auto-calibrage et un circuit intégré de commande d'un tel stimulateur ; l'invention propose également un procédé de calibrage d'un système de stimulation électrique.

L'invention s'applique en particulier, mais pas exclusivement, aux stimulateurs électriques implantables, notamment pour la stimulation cérébrale profonde, la stimulation rétinienne, la stimulation cochléaire, la stimulation du nerf vague, etc.

Les stimulateurs électriques conventionnels délivrent des impulsions de courant ou de tension dont la forme est prédéterminée.

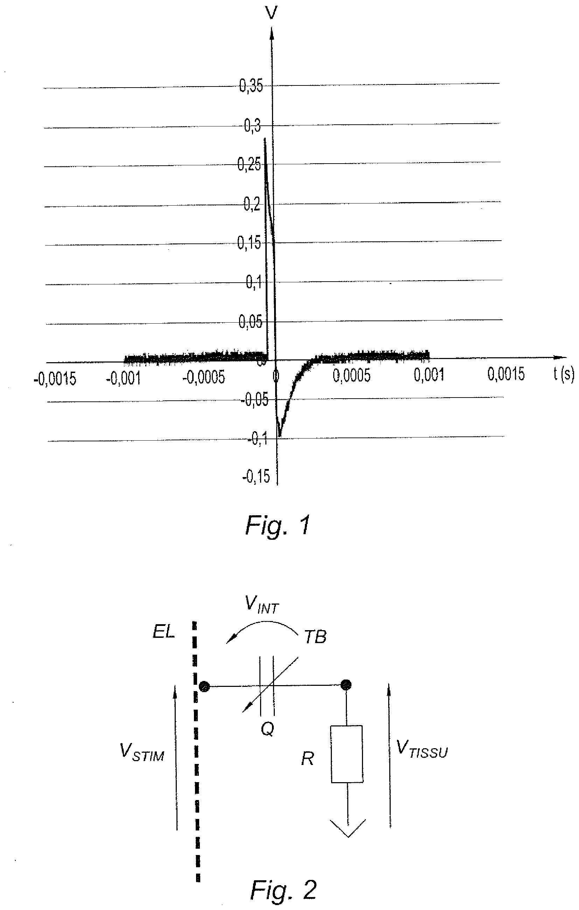

Les stimulateurs cérébraux utilisés dans la pratique clinique utilisent principalement des impulsions dites « de Lilly » (voir la figure 1), introduites par le Dr John C Lilly dans sa publication « Injury and Excitation by Electric Currents - A. The Balanced Pulse-Pair Waveform », Chapitre 6 du livre « Electrical Stimulation of the Brain », édité par Daniel E. Sheer, Ed., Univ. of Texas Press for Hogg Foundation for Mental Health, Austin, Texas, pages 60-64,1961. IONESCU C ET AL " Activating Paralyzed Muscles using Adaptive Control and a Neuroprosthetic Technique" IEEE International Conference on Automation, Quality and Testing, Robotics, 2006 décrit un système de stimulation musculaire utilisant un régulateur auto-adaptatif en boucle fermée. Dans la stimulation cérébrale chronique (implantation à long terme) se pose le problème de rester au dessus du seuil de stimulation sans endommager les tissus. Les impulsions de Lilly se sont montrées particulièrement adaptées à atteindre cet objectif. Si le choix de la forme du signal est figé, le chirurgien doit adapter son amplitude ainsi que sa fréquence de répétition afin d'obtenir les signes cliniques attendus. Par exemple, pour un stimulateur cérébral profond, les paramètres de stimulation habituels pour le traitement de la maladie de Parkinson sont : -3V pour l'amplitude de pic et 130Hz pour la fréquence de répétition des impulsions de Lilly.

Pour la stimulation rétinienne, bien que cette technique soit encore peu développée et beaucoup moins au point que les techniques de stimulation cérébrale profonde, les signaux les plus utilisés sont des impulsions carrées biphasiques, comprenant une phase positive suivie d'une négative ou vice-versa. Les valeurs de tensions sont généralement comprises entre 90 mV et 10,5 V, et les valeurs de courant sont généralement comprises entre 7 nA - 25mA ; les durées sont généralement comprises entre 1 μs et 10 ms.

Un inconvénient de l'art antérieur est que l'impulsion effectivement reçue par le tissu à stimuler ne correspond pas exactement à celle qui est délivrée par le stimulateur. Dans le cas d'impulsions de tension, en effet, le tissu biologique et son interface avec le stimulateur (et plus précisément ses électrodes) se comportent comme un filtre, qui déforme l'impulsion. Par ailleurs, les paramètres de ce filtre peuvent évoluer dans le temps, selon l'environnement biologique et chimique dans lequel se trouvent les électrodes. Par exemple une des causes de cette ou ces modifications est le développement de tissu fibreux ou de gliose au tour de l'électrode ainsi que la corrosion des matériaux constitutifs de l'implant. Cela a plusieurs conséquences indésirable : l'efficacité thérapeutique de la stimulation peut être compromise (dans certains cas les impulsions modifiées peuvent même provoquer des lésions), et la consommation énergétique du stimulateur peut être inutilement élevée. Dans le cas d'impulsions de courant, une augmentation de l'impédance vue par les électrodes peut conduire à une saturation des étages de sortie du stimulateur,

L'invention vise à pallier les inconvénients précités de l'art antérieur.

Un premier objet de l'invention est un système de stimulation électrique comprenant : un moyen pour générer au moins un signal électrique à appliquer à un tissu biologique à stimuler et pour mesurer une réponse dudit tissu biologique audit ou à chaque dit signal électrique ; un moyen de calcul pour estimer, à partir de la connaissance dudit ou de chaque dit signal électrique et de la réponse correspondante dudit tissu biologique, au moins un paramètre d'un modèle électrique dudit tissu biologique et de son interface avec ledit système de stimulation électrique et pour déterminer, à l'aide dudit modèle, au moins un paramètre d'une impulsion de stimulation à appliquer audit tissu biologique au moyen dudit système de stimulation électrique ; et un moyen pour générer une dite impulsion de stimulation à appliquer audit tissu biologique.

Ce système de stimulation électrique peut être de type implantable, et notamment être choisi parmi : un appareil implantable de stimulation cérébrale profonde ; un appareil implantable de stimulation rétinienne ; un appareil implantable de stimulation du nerf vague ; un appareil implantable de stimulation corticale et un appareil implantable de stimulation musculaire.

Selon un mode de réalisation particulièrement avantageux, ledit moyen de calcul peut être adapté (c'est-à-dire conçu, configuré et/ou programmé) pour estimer une fonction de transfert dudit modèle électrique, et pour déterminer au moins un paramètre de ladite impulsion de stimulation à appliquer au tissu biologique à partir de ladite fonction de transfert.

Plus particulièrement, selon un mode de réalisation préféré, ledit moyen de calcul peut être adapté pour déterminer au moins un paramètre de ladite impulsion de stimulation à appliquer au tissu biologique à partir de ladite fonction de transfert et d'une impulsion de référence prédéterminée.

Selon un autre mode de réalisation préféré, le moyen de calcul peut être adapté pour déterminer ledit paramètre de l'impulsion de stimulation de telle sorte qu'au moins un paramètre de l'impulsion effectivement appliquée au tissu biologique prenne une valeur égale à une valeur de référence, avec une marge d'erreur prédéfinie. Cette valeur de référence peut correspondre notamment à un paramètre correspondant (par exemple, une tension de pic) d'une impulsion de référence prédéfinie.

Un autre objet de la description est un circuit intégré de commande d'un système de stimulation électrique comprenant : un moyen pour générer au moins un signal électrique à appliquer à un tissu biologique à stimuler et pour mesurer une réponse dudit tissu biologique audit ou à chaque dit signal électrique ; et un moyen de calcul pour estimer, à partir de la connaissance dudit ou de chaque dit signal électrique et de la réponse correspondante dudit tissu biologique, au moins un paramètre d'un modèle électrique dudit tissu biologique et de son interface avec ledit système de stimulation électrique et pour déterminer, à l'aide dudit modèle, au moins un paramètre d'une impulsion de stimulation à appliquer audit tissu biologique au moyen dudit système de stimulation électrique.

Selon un mode de réalisation particulièrement avantageux, ledit moyen de calcul peut être adapté (c'est-à-dire conçu, configuré et/ou programmé) pour estimer une fonction de transfert dudit modèle électrique, et pour déterminer au moins un paramètre de ladite impulsion de stimulation à appliquer au tissu biologique à partir de ladite fonction de transfert.

Plus particulièrement, selon un mode de réalisation préféré, ledit moyen de calcul peut être adapté pour déterminer au moins un paramètre de ladite impulsion de stimulation à appliquer au tissu biologique à partir de ladite fonction de transfert et d'une impulsion de référence prédéterminée.

Selon un autre mode de réalisation préféré, le moyen de calcul peut être adapté pour déterminer ledit paramètre de l'impulsion de stimulation de telle sorte qu'au moins un paramètre de l'impulsion effectivement appliquée au tissu biologique prenne une valeur égale à une valeur de référence, avec une marge d'erreur prédéfinie. Cette valeur de référence peut correspondre notamment à un paramètre correspondant (par exemple, une tension de pic) d'une impulsion de référence prédéfinie.

Un tel circuit intégré peut comprendre également un moyen pour générer une dite impulsion de stimulation à appliquer audit tissu biologique.

Encore un autre objet de l'invention est un procédé de calibrage d'un système de stimulation électrique d'un tissu biologique, comprenant les étapes consistant à :

    a. utiliser ledit système de stimulation électrique pour appliquer au moins un signal électrique audit tissu biologique et pour mesurer une réponse dudit tissu biologique audit ou à chaque dit signal électrique ;
    b. à partir de la connaissance dudit ou de chaque dit signal électrique et de la réponse correspondante dudit tissu biologique, estimer de manière automatique au moins un paramètre d'un modèle électrique dudit tissu biologique et de son interface avec ledit système de stimulation électrique ; et
    c. utiliser ledit modèle électrique pour déterminer de manière automatique au moins un paramètre d'une impulsion de stimulation à appliquer audit tissu biologique au moyen dudit système de stimulation électrique.

Selon différents modes de réalisation :

- Un tel procédé peut comporter l'utilisation d'électrodes de stimulation dudit système de stimulation électrique pour mettre en oeuvre ladite étape a.
- L'étape c. peut comprendre l'utilisation dudit modèle électrique pour estimer une fonction de transfert du tissu biologique et de son interface avec ledit système de stimulation électrique, et la détermination d'au moins un paramètre de ladite impulsion de stimulation à appliquer au tissu biologique à partir de ladite fonction de transfert.
- L'étape c. peut comprendre la détermination dudit paramètre de l'impulsion de stimulation de telle sorte qu'au moins un paramètre de l'impulsion effectivement appliquée au tissu biologique prenne une valeur égale à une valeur de référence, avec une marge d'erreur prédéfinie.
- Lesdites étapes b. et c. peuvent être mises en oeuvre par des moyens de calcul dudit système de stimulation électrique.

D'autres caractéristiques, détails et avantages de l'invention ressortiront à la lecture de la description faite en référence aux dessins annexés donnés à titre d'exemple et qui représentent, respectivement :

- La figure 1, une impulsion électrique de type « de Lilly » ;
- La figure 2, un circuit électrique équivalent modélisant un tissu biologique et son interface avec une électrode de stimulation ;
- Les figures 3A - 3B, le module et la phase du spectre d'impédance du système constitué par le cerveau d'un singe et son interface avec une électrode millimétrique de stimulation cérébrale profonde, mesuré *in vivo,* et d'un filtre électrique RC modélisant cette interface ;

- Les figures 4A - 4B le module et la phase du spectre d'impédance du système constitué par la rétine d'un rat et son interface avec une matrice de microélectrodes, mesuré *in vivo*, et d'un filtre électrique RC modélisant cette interface ;
- La figure 5, la déformation d'une impulsion carrée induite par le système constitué par un tissu biologique et son interface avec une microélectrode rétinienne ;
- Les figures 6A et 6B, la déformation d'une impulsion « de Liliy » induite par le système constitué par un tissu biologique et son interface avec, respectivement, une électrode de stimulation cérébrale profonde et une microélectrode rétinienne ;
- La figure 7, le schéma fonctionnel d'un stimulateur électrique selon un mode de réalisation de l'invention ;
- La figure 8, la détermination d'une fonction de transfert entre un signal X et un signal Y ;
- Les figures 9A et 9B, un premier algorithme de détermination de la durée et/ou de la forme d'une impulsion de stimulation ; et
- La figure 10, un deuxième algorithme de détermination de la durée et/ou de la forme d'une impulsion de stimulation.

Des modélisations numériques et des mesures d'impédance *in vivo* permettent de montrer que le système constitué par un tissu biologique et son interface avec une électrode se comporte comme un filtre qui peut être modélisé par un circuit électrique équivalent comprenant des éléments réactifs et résistifs. Le modèle le plus simple est constitué par un circuit RQ série formant un filtre passe-haut, comme illustré sur la figure 2. L'élément R modélise la résistance électrique du tissu ; l'élément Q est un « élément à phase constante », qui modélise le comportement diélectrique imparfait de l'interface. Un élément à phase constante est un élément passif dont l'impédance (complexe) $Z_{CPE}$ présente un angle de phase indépendant de la fréquence, égal à $-(90*n)°$ :

$$Z_{CPE} = \frac{1}{Q_0 (i\omega)^n} = \frac{1}{Q_0 \omega^n} e^{-\frac{\pi}{2} ni}$$

où $\omega$ est la pulsation du signal, $Q_0 = |Z_{CPE}|^{-1}$ à $\omega = 1$ rad/s et $0 \leq n \leq 1$. Sur la figure 2, $V_{STIM}$ est la tension de stimulation appliquée par le stimulateur électrique (on suppose l'utilisation d'un signal de tension, ce qui est le cas le plus courant) à travers l'électrode EL et $V_{TISSU}$ la tension réellement appliquée au tissu TB ; $V_{INT}$ est la chute de tension sur l'interface, de telle sorte que $V_{STIM} = V_{INT} + V_{TISSU}$.

Le résisteur et le condensateur peuvent être considérés comme des cas limites d'éléments à phase constante : $n=0$ dans le cas du résisteur, ce qui implique un angle de phase nul ; $n=1$ dans le cas du condensateur, ce qui implique un angle de phase égal à $-90°$. Dans le cas où l'élément à phase constante est une capacité ordinaire, le circuit de la figure 2 devient un filtre RC série.

Les valeurs de R, de $Q_0$ (ou C, si on fixe $n=1$) et, le cas échéant, de n, peuvent être ajustées de manière à reproduire aussi fidèlement que possible - par exemple selon un critère de moindre erreur quadratique sur une plage de fréquences d'intérêt - la valeur de l'impédance complexe du système interface-tissu. Par exemple :

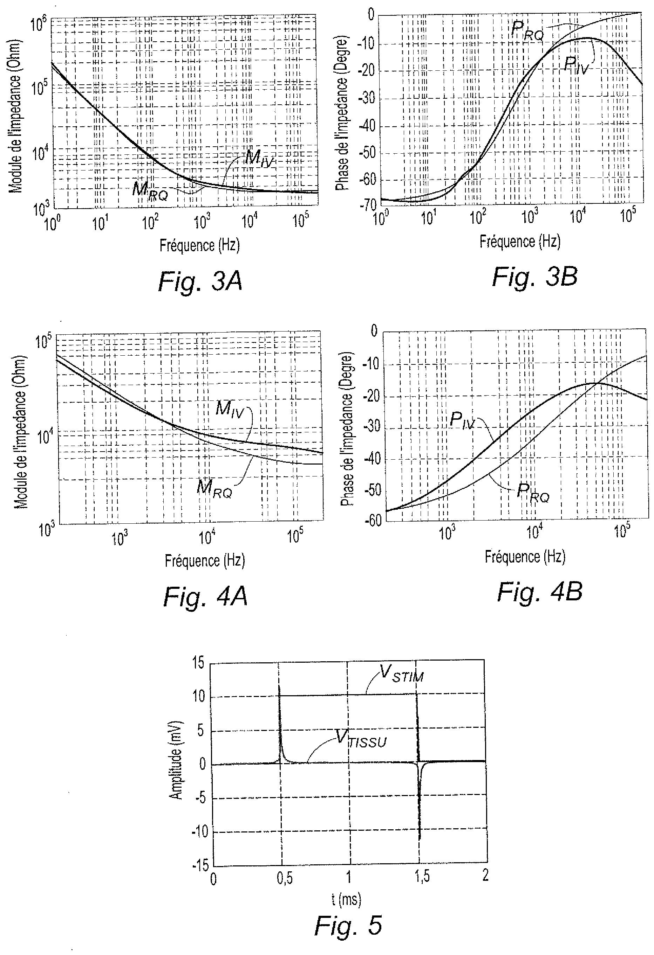

- Sur la figure 3A (respectivement : 3B) la courbe $M_{IV}$ (respectivement $P_{IV}$) représente le module (respectivement : l'angle de phase) de l'impédance d'une électrode millimétrique de stimulation cérébrale profonde (Electrode Medtronic, voir 3389 http://professional.medtronic.com/pt/neuro/dbs-md/prod/dbs-lead-model-3389/index.htm) avec le tissu cérébral d'un singe (macaque), mesurée *in vivo* en fonction de la fréquence ; la courbe $M_{RQ}$ (respectivement $P_{RQ}$) représente le module (respectivement : l'angle de phase) de l'impédance d'entrée du filtre RC qui modélise le mieux ce système. On trouve $R=1665\ \Omega$, $Q_0=429$ nF.s$^n$ avec $n=0,76$. Dans le cas de l'élément à phase constante, l'unité de $Q_0$ est donnée en [F] (Farad) par [s]$^{n-1}$. Ainsi le produit $Q_0.\omega^n$ est exprimé en [F]·[s]$^{n-1}$·[s]$^{-n}$ soit [F]·[s]$^{-1}$ homogène au produit $C·\omega$ dans le cas d'une capacité.
- Sur la figure 4A (respectivement : 4B) la courbe $M_{IV}$ (respectivement $P_{IV}$) représente le module (respectivement : l'angle de phase) de l'impédance d'une matrice de microélectrodes rétiniennes implantée sous la rétine d'un rat, mesurée *in vivo* en fonction de la fréquence ; la courbe $M_{RQ}$ (respectivement $P_{RQ}$) représente le module (respectivement : l'angle de phase) de l'impédance d'entrée du filtre RC qui modélise le mieux ce système. On trouve $R=4087\Omega$, $Q_0= 13,37$ nF·s$^{n-1}$, avec $n=0,83$.

La déformation induite par le tissu et son interface avec l'électrode est illustrée sur la figure 5 dans le cas d'une impulsion de stimulation de type créneau de tension, avec une amplitude de 10 mV et une durée de 200 $\mu$s, appliquée entre deux électrodes micrométriques circulaire, présentant un rayon de 50 $\mu$m, espacées de 100 $\mu$m (bord à bord) ; il s'agit là de valeurs typiques pour une stimulation rétinienne. Pour ce type de stimulation, on utilise par exemple une matrice de 9 électrodes telles que définies ci-dessus, réparties selon trois rangées de trois électrodes. Les paramètres du filtre de modélisation sont : $R=17{,}7k\Omega$, $Q_0 = 3{,}35$ nF·s$^{-1}$, avec $n=0{,}801$.

On voit clairement que l'impulsion $V_{TISSU}(t)$ effectivement appliquée au tissu rétinien est très différente de celle, $V_{STIM}(t)$, générée par le stimulateur. En particulier elle est beaucoup plus courte, car $V_{TISSU}(t)=0$ pour $t>60$ $\mu$s environ, alors que $V_{STIM}$ se maintient à une valeur « haute » pendant 200 $\mu$s,

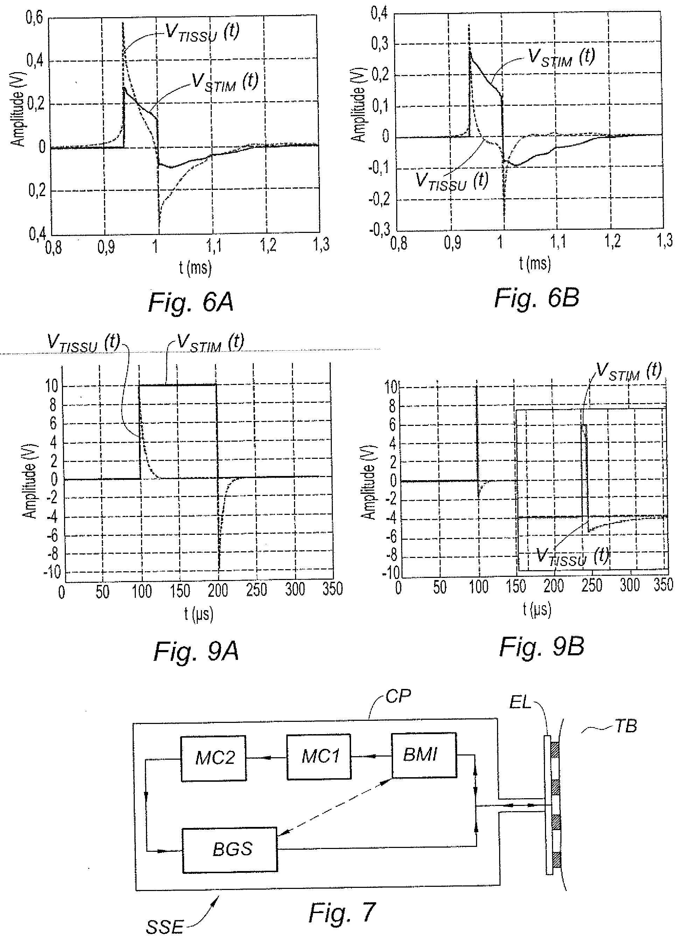

La figure 6A montre qu'une impulsion de tension « de Lilly » est déformée par une électrode millimétrique, du type couramment utilisé en stimulation cérébrale profonde ($R=17$ $\Omega$ ; $Q_0=33{,}5$ $\mu$F·s$^{-1}$), mise en oeuvre sur un singe. La figure 6B montre que la déformation est encore plus importante dans le cas d'une microélectrode d'une matrice de stimulation rétinienne, implantée sur un rat, le milieu étant alors modélisé par les paramètres de l'électrode : $R=17{,}7$ k$\Omega$ ; $Q_0=3{,}35$ nF s$^{-1}$. Ainsi, pour une même impulsion de stimulation, le signal effectivement appliqué au tissu ($V_{TISSU}(t)$) varie en fonction du milieu biologique. Cela peut nuire à l'efficacité thérapeutique de la stimulation.

Une idée à la base de l'invention consiste à tenir compte du filtrage introduit par le système constitué par un tissu biologique et son interface avec une électrode de stimulation pour calibrer un stimulateur électrique.

Plus précisément, conformément à l'invention, un stimulateur électrique est utilisé pour caractériser le système avec lequel il interagit - tissu et interface - déterminer les paramètres d'un modèle électrique de ce système (auto-calibrage) et utiliser ces paramètres pour optimiser la forme et/ou la durée des impulsions de stimulation. De cette manière, il est possible d'adapter la forme du signal de stimulation au filtre identifié, de manière à maitriser la forme et l'amplitude du signal effectivement appliqué au tissu.

La figure 7 montre un schéma fonctionnel d'un stimulateur électrique SSE selon un mode de réalisation de l'invention. Ce stimulateur comprend au moins une électrode - ou matrice d'électrodes - de stimulation EL, destinée à entrer en contact avec un tissu biologique TB à stimuler, et un circuit électronique de pilotage CP pour, entre autres, générer les impulsions de stimulation qui seront appliquées au tissu TB par l'intermédiaire de l'électrode ou des électrodes EL.

Comme discuté plus haut, l'électrode ou matrice d'électrodes EL peut présenter une forme/géométrie et des dimensions très différentes en fonction de l'application visée, allant d'une électrode unique présentant une surface de quelques millimètres carrés pour la stimulation cérébrale profonde à une matrice de plusieurs dizaines ou centaines de microélectrodes planaires ou tridimensionnelles, présentant des surfaces unitaires de l'ordre de la dizaine ou de la centaine de micromètres carrés, pour la stimulation rétinienne ou cochléaire. Dans la suite on considérera une matrice d'électrodes planaires circulaires ayant chacune un rayon de 50 $\mu$m, avec un espacement inter-électrodes (bord à bord) de 100 $\mu$m.

Le tissu biologique TB est un milieu salin, contenant des ions Na$^+$ et Ca$^+$ à une concentration d'environ 138 mM, ainsi que des protéines et des cellules sa conductivité est estimée empiriquement à 1,4178 S/m.

Le circuit électronique de pilotage du stimulateur comprend notamment :

- un bloc de mesure d'impédance BMI, pour générer au moins un signal électrique à appliquer au tissu TB, typiquement au moyen de l'électrode/matrice d'électrodes EL, et pour mesurer (typiquement au moyen de cette même électrode ou matrice d'électrodes) une réponse dudit tissu biologique audit ou à chaque dit signal électrique ;
- un premier moyen de calcul MC1 pour ajuster les paramètres d'un modèle électrique (RQ série, RC série ou plus élaboré) aux mesures effectuées par le bloc BMI ;
- un deuxième moyen de calcul MC2 pour déterminer la forme et/ou l'intensité et/ou la durée d'une impulsion de stimulation en fonction des valeurs des paramètres déterminées par le bloc MC1 ; et
- un bloc générateur de signaux BGS pour générer cette impulsion de stimulation ; le bloc BGS peut également coopérer avec le bloc BMI pour générer les signaux à appliquer au tissu TB lors de la mesure d'impédance.

Ces blocs ne sont pas nécessairement physiquement distincts les uns des autres. Par exemple, les différentes opérations de calcul, ou certaines d'entre elles, peuvent être mises en oeuvre par différents circuits logiques câblés, ou bien par un processeur unique programmé de manière opportune. La totalité, ou seulement certains, de ces blocs peuvent être réunis dans un circuit intégré dédié (« ASIC »), numérique ou hybride numérique - analogique. L'ensemble du système de stimulation n'est pas nécessairement implanté avec les électrodes. Seule la partie effectrice (bloc générateur de signaux) peut par exemple être implémentée dans un ASIC et le reste des fonctions de traitement de signal et de commande peut être déporté sur un circuit externe (FPGA, microcontrôleur ...).

Le bloc BMI peut réaliser une spectroscopie d'impédance en faisant passer un faible courant sinusoïdal entre deux électrodes de stimulation (configuration bipolaire) et en mesurant la tension à leurs bornes, cette mesure étant répétée pour plusieurs valeurs de fréquence du courant sinusoïdal.

En variante, les électrodes peuvent être utilisés pour appliquer au tissu une impulsion de tension et mesurer sa réponse en courant (ou inversement : stimuler le tissu en courant et mesurer sa réponse en tension). Ensuite l'impédance est estimée par différentes techniques de traitement de signal ; on peut se rapporter à ce propos à l'article de M. Min et al. « Broadband excitation for short-time impedance spectroscope », Physiol. Meas. 29 (2008) S185-S192.

L'impédance ainsi mesurée est utilisée par le premier moyen de calcul MC1 pour ajuster les paramètres d'un modèle électrique (circuit électrique équivalent) du système constitué par l'interface électrode/tissu et le tissu lui-même. Ensuite, ledit premier moyen de calcul détermine la fonction de transfert H de ce circuit électrique équivalent.

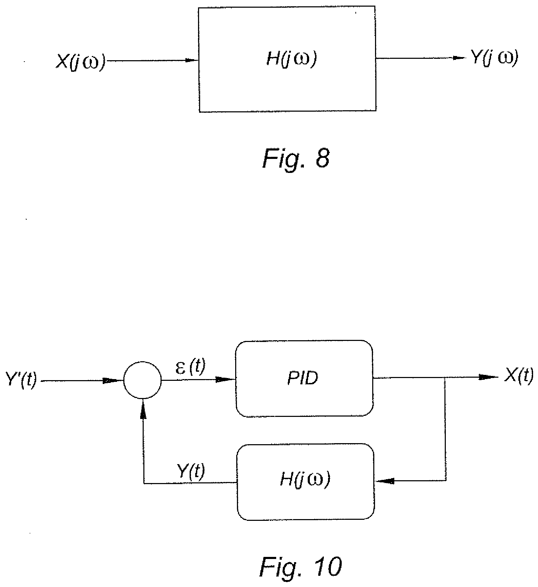

Par fonction de transfert, on entend la fonction reliant la tension $V_{TISSU}$ (ou le courant) appliqué au tissu à la tension $V_{STIM}$ (ou au courant) de stimulation. Plus précisément, en référence à la figure 8, si X représente le signal d'entrée de la fonction de transfert (tension ou courant de stimulation $V_{STIM}$) et Y représente le signal de sortie de la fonction de transfert (tension ou courant appliqué au tissu $V_{TISSU}$), on a :

$$H(j\omega) = \frac{Y(j\omega)}{X(j\omega)} = \frac{V_{TISSU}(j\omega)}{V_{STIM}(j\omega)}$$

$X(j\omega)$ et $Y(j\omega)$ désignant respectivement les transformées de Laplace des expressions temporelles de X et Y, respectivement X(t) et Y(t) telles que :

$$X(t) = L^{-1}\left[X(j\omega)\right]$$

$$Y(t) = L^{-1}\left[Y(j\omega)\right]$$

L désignant l'opérateur de la transformée de Laplace.

Il est également possible d'utiliser une configuration de mesure dite monopolaire, avec une seule électrode de stimulation et un retour passif « à l'infini », c'est-à-dire une électrode de grande taille (surface au moins dix fois supérieure à celle de l'électrode de stimulation), appelée « contre électrode », située à une distance très supérieure au champ d'action de l'électrode de stimulation. Ainsi, cette « contre-électrode » n'influe pas sur la forme du champ électrique généré par l'électrode de travail.

Ainsi, d'une façon générale, le moyen de calcul MC1 permet d'estimer une fonction de transfert H, reliant le signal électrique généré par le stimulateur (par exemple $V_{STIM}$) et le signal électrique réellement appliqué au milieu (par exemple $V_{TISSU}$).

A l'aide de cette fonction de transfert H, le deuxième moyen de calcul MC2 détermine une forme (par exemple une amplitude et/ou une durée) de l'impulsion de stimulation. Cela peut être effectué sur la base de plusieurs algorithmes, dont des exemples sont donnés ci-dessous.

Un premier algorithme, illustré par les figures 9A et 9B, simule ce même circuit équivalent et diminue de plus en plus la largeur de l'impulsion $V_{STIM}$. Celle-ci ressemble alors de plus en plus au signal d'origine - quelle que soit la forme de cette dernière (carrée dans le cas considéré ici). L'algorithme s'arrête lorsque la forme du signal $V_{TISSU}$(t) simulé est jugé suffisamment proche de $V_{STIM}$(t) par l'opérateur. Par suffisamment proche, on entend qu'un paramètre du signal $V_{TISSU}$(t), par exemple l'amplitude maximum et/ou l'amplitude minimum correspond à celle de celui du signal $V_{STIM}$(t) à un pourcentage près, prédéterminé par l'opérateur. La figure 9A se rapporte au cas d'une impulsion carrée de durée « longue », pour laquelle $V_{TISSU}$(t) est très différente de $V_{STIM}$(t), tandis que la figure 9B illustre le cas d'une impulsion carrée beaucoup plus brève, pour laquelle on peut considérer que $V_{TISSU}$(t)≈$V_{STIM}$(t).

Ainsi, les blocs BMI, MC1 et MC2 réalisent un calibrage - ou plutôt un auto-calibrage - du stimulateur. Ce calibrage n'est pas réalisée une fois pour toutes mais doit être répété régulièrement, car les propriétés du tissu et de l'interface sont évolutives.

Le bloc BGS est conventionnel. Il reçoit en entrée le ou les paramètres déterminés par le bloc MC2 et il génère les impulsions de stimulation correspondantes. Ce bloc peut également servir pour générer les impulsions utilisées pour la caractérisation du tissu biologique ; cela nécessite une coopération directe avec le bloc BMI, représentée schématiquement sur la figure 7 par une double flèche en trait pointillé.

Un deuxième algorithme estime la fonction de transfert inverse $H_{INV}$ de la fonction de transfert H du milieu. Ainsi, si l'on souhaite appliquer au milieu un signal Y' ($V'_{TISSU}$) prédéterminé, dit signal de consigne, l'algorithme permet d'établir le signal X ($V_{STIM}$) à appliquer, tel que $X(j\omega) = H_{inv}(j\omega).Y'(j\omega)$. Le signal effectivement appliqué au tissu est alors :

$$Y(j\omega) = H(j\omega)X(j\omega) = H(j\omega)\, H_{INV}(j\omega)Y'(j\omega) \approx Y'(j\omega)$$

Ainsi, connaissant le signal à appliquer au tissu $V'_{TISSU}$ (signal de référence ou de consigne), un tel signal étant déterminé a priori par l'utilisateur, et connaissant la fonction de transfert du milieu H, cette dernière étant déterminée comme indiqué précédemment, on estime la fonction de transfert inverse $H_{inv}$ et on détermine le signal de stimulation $V_{STIM}$, qui permet de générer un signal $V_{TISSU} = V'_{TISSU}$

Cependant, l'estimation de $H_{inv}$ peut s'avérer délicate, du fait de la non linéarité de la réponse électrique du milieu et des différents bruits affectant les signaux, bruits d'origine biologique et/ou électronique. On détermine généralement le signal de stimulation X, connaissant le signal de consigne Y', en utilisant l'estimation de la fonction de transfert H. Cette détermination peut par exemple être effectuée en mettant en oeuvre une boucle de régulation itérative dont l'objectif est de générer un signal de commande X (permettant d'appliquer au tissu un signal approchant le signal de consigne Y'), connaissant la fonction de transfert H. Au-delà d'une certaine durée, qui correspond au temps d'établissement d'un régime stationnaire, le correcteur délivre un signal X tel que le signal Y appliqué au tissu (ou l'un de ses paramètres) corresponde au signal Y' de consigne (ou l'un de ses paramètres) à une erreur près.

Une telle boucle de rétroaction peut mettre en oeuvre un régulateur PID (Proportionnel Intégral Dérivé), comme cela est représenté sur la figure 10.

L'invention s'applique dans le cas où les impulsions de stimulation sont contrôlées en tension, car c'est dans ce mode de fonctionnement que les déformations introduites par le système tissu/interface sont les plus importantes. Elle s'applique également au cas d'une impulsion en courant contrôlé, le stimulus appliqué au tissu est identique à celui généré par le stimulateur. Cependant, la tension générée est proportionnelle à l'impédance du tissu et/ou susceptible d'amener à la saturation des étages de sortie du stimulateur. Or si cette impédance est très forte (de l'ordre de quelque MΩ à basse fréquence), un faible courant peut générer une très forte tension, nuisible pour les tissus. Dans ces conditions, la caractérisation du tissu par le stimulateur permet de déterminer le courant maximal admissible, et d'éviter les lésions induites par des valeurs trop élevées de tension.

## Revendications

1. Stimulateur électrique (SSE) comprenant au moins :

    - une électrode, ou matrice d'électrodes, de stimulation (EL) destinée à entrer en contact avec un tissus biologique (TB) à stimuler ;
    - un circuit électronique de pilotage (CP) pour générer des impulsions de stimulation qui seront appliquées au tissu (TB) par l'intermédiaire de l'électrode ou des électrodes (EL) ;
    - un moyen (BMI) pour générer au moins un signal électrique à appliquer à un tissu biologique à stimuler (TB) et pour mesurer une réponse dudit tissu biologique audit ou à chaque dit signal électrique ;
    - un moyen (BGS) pour générer une dite impulsion de stimulation $(X, V_{STIM})$ à appliquer audit tissu biologique ;

    **caractérisé en ce qu'**il comprend :

    - un moyen de calcul (MC1, MC2) pour estimer, à partir de la connaissance dudit ou de chaque dit signal électrique et de la réponse correspondante dudit tissu biologique, au moins un paramètre d'un modèle électrique dudit tissu biologique et de son interface avec la ou les électrodes et pour déterminer, à l'aide dudit modèle, au moins un paramètre d'une impulsion de stimulation à appliquer audit tissu biologique au moyen dudit stimulateur électrique et adapté pour estimer une fonction de transfert (H) dudit modèle électrique, la dite fonction de transfert reliant la tension (VTISSU) ou le courant appliqué au tissu à la tension (VSTIM) ou au courant de stimulation, et pour déterminer au moins un paramètre de ladite impulsion de stimulation à appliquer au tissu biologique à partir de ladite fonction de transfert et d'une impulsion de référence prédéterminée (Y', V'$_{TISSU}$).

2. Stimulateur électrique selon l'une des revendications précédentes dans lequel ledit moyen de calcul est adapté pour déterminer ledit paramètre de l'impulsion de stimulation de telle sorte qu'au moins un paramètre de l'impulsion effectivement appliquée au tissu biologique (Y, V$_{TISSU}$) prenne une valeur égale à une valeur de référence, avec une marge d'erreur prédéfinie.

3. Stimulateur électrique selon l'une des revendications précédentes, de type implantable.

4. Stimulateur électrique selon la revendication 3, choisi parmi :

    - un appareil implantable adapté pour la stimulation cérébrale profonde ;
    - un appareil implantable adapté pour la stimulation rétinienne ;
    - un appareil implantable adapté pour la stimulation du nerf vague ;
    - un appareil implantable adapté pour la stimulation corticale et
    - un appareil implantable adapté pour la stimulation musculaire.

**5.** Procédé de calibrage d'un stimulateur électrique d'un tissu biologique (TB) selon l'une des revendications 1 à 4, comprenant les étapes consistant à :

a. utiliser ledit stimulateur électrique (SSE) pour appliquer au moins un signal électrique audit tissu biologique et pour mesurer une réponse dudit tissu biologique audit ou à chaque dit signal électrique ;

b. à partir de la connaissance dudit ou de chaque dit signal électrique et de la réponse correspondante dudit tissu biologique, estimer de manière automatique au moins un paramètre d'un modèle électrique dudit tissu biologique et de la ou les électrodes du stimulateur électrique ; et

c. utiliser ledit modèle électrique pour déterminer de manière automatique au moins un paramètre d'une impulsion de stimulation ($V_{STIM}$) à appliquer audit tissu biologique au moyen dudit stimulateur électrique et pour estimer une fonction de transfert du tissu biologique et de son interface avec la ou les électrodes du stimulateur électrique, la dite fonction de transfert reliant la tension (VTISSU) ou le courant appliqué au tissu à la tension (VSTIM) ou au courant de stimulation, et déterminer au moins un paramètre de ladite impulsion de stimulation à appliquer au tissu biologique à partir de ladite fonction de transfert.

**6.** Procédé selon la revendication 5, dans lequel ladite étape c. comprend la détermination dudit paramètre de l'impulsion de stimulation de telle sorte qu'au moins un paramètre de l'impulsion effectivement appliquée au tissu biologique prenne une valeur égale à une valeur de référence, avec une marge d'erreur prédéfinie.

**7.** Procédé selon l'une des revendications 5 ou 6, dans lequel lesdites étapes b. et c. sont mises en oeuvre par des moyens de calcul dudit stimulateur électrique.


**Patentansprüche**

**1.** Elektrischer Stimulator (SSE), der mindestens Folgendes umfasst:

- eine Elektrode oder Elektrodenmatrix zur Stimulation (EL), die dazu bestimmt ist, mit einem zu stimulierenden biologischen Gewebe (TB) in Berührung zu kommen;
- eine elektronische Steuerschaltung (CP) zum Erzeugen von Stimulationsimpulsen, die mittels der Elektrode oder Elektroden (EL) an das Gewebe (TB) angelegt werden;
- ein Mittel (BMI) zum Erzeugen von mindestens einem elektrischen Signal, das an ein zu stimulierendes biologisches Gewebe (TB) anzulegen ist, und zum Messen einer Reaktion des biologischen Gewebes auf das oder jedes elektrische Signal;
- ein Mittel (BGS) zum Erzeugen eines an das biologische Gewebe anzulegenden Stimulationsimpulses (X, $V_{STIM}$);

**dadurch gekennzeichnet, dass** er Folgendes umfasst:

- ein Rechenmittel (MC1, MC2) zum Schätzen von mindestens einem Parameter eines elektrischen Modells des biologischen Gewebes und seiner Grenzfläche mit der oder den Elektroden ausgehend von der Kenntnis von dem oder jedem elektrischen Signal und der entsprechenden Reaktion des biologischen Gewebes, und zum Bestimmen mindestens eines Parameters eines mittels des elektrischen Stimulators an das biologische Gewebe anzulegenden Stimulationsimpulses mittels des Modells, und angepasst ist, um eine Übertragungsfunktion (H) des elektrischen Modells zu schätzen, wobei die Übertragungsfunktion die/den an das Gewebe angelegte/n Spannung (VTISSU) oder Strom mit der/dem Stimulationsspannung (VSTIM) oder -strom verbindet, und um mindestens einen Parameter des an das biologische Gewebe anzulegenden Stimulationsimpulses ausgehend von der Übertragungsfunktion und einem vorbestimmten Bezugsimpuls (Y', V'$_{TISSU}$) zu bestimmen.

**2.** Elektrischer Stimulator nach einem der vorhergehenden Ansprüche, wobei das Rechenmittel angepasst ist, um den Stimulationsimpulsparameter derart zu bestimmen, dass mindestens ein Impulsparameter, der effektiv an das biologische Gewebe (Y, $V_{TISSU}$) angelegt wird, einen Wert annimmt, der gleich einem Bezugswert mit einer vordefinierten Fehlermarge ist.

**3.** Elektrischer Stimulator nach einem der vorhergehenden Ansprüche vom implantierbaren Typ.

**4.** Elektrischer Stimulator nach Anspruch 3, der unter Folgendem ausgewählt ist:

- einer implantierbaren Vorrichtung, die für die Tiefenhirnstimulation angepasst ist;
- einer implantierbaren Vorrichtung, die für die Netzhautstimulation angepasst ist;
- einer implantierbaren Vorrichtung, die für die Stimulation des Vagusnervs angepasst ist;
- einer implantierbaren Vorrichtung, die für die kortikale Stimulation angepasst ist; und
- einer implantierbaren Vorrichtung, die für die muskuläre Stimulation angepasst ist.

5. Verfahren zur Kalibrierung eines elektrischen Stimulators für ein biologisches Gewebe (TB) nach einem der Ansprüche 1 bis 4, das die Schritte umfasst, die aus Folgendem bestehen:

   a. Verwenden des elektrischen Stimulators (SSE) zum Anlegen mindestens eines elektrischen Signals an das biologische Gewebe und zum Messen einer Reaktion des biologischen Gewebes auf das oder jedes elektrische Signal;
   b. ausgehend von der Kenntnis des oder der elektrischen Signale und der entsprechenden Reaktion des biologischen Gewebes, automatisches Schätzen von mindestens einem Parameter eines elektrischen Modells des biologischen Gewebes und der Elektrode oder Elektroden des elektrischen Stimulators; und
   c. Verwenden des elektrischen Modells zum automatischen Bestimmen von mindestens einem Parameter eines mittels des elektrischen Stimulators an das biologische Gewebe anzulegenden Stimulationsimpulses ($V_{STIM}$) und zum Schätzen einer Übertragungsfunktion des biologischen Gewebes und seiner Grenzfläche mit der oder den Elektroden des elektrischen Stimulators, wobei die Übertragungsfunktion die/den an das Gewebe angelegte/n Spannung (VTISSU) oder -strom mit der/dem Stimulationsspannung (VSTIM) oder -strom verbindet, und Bestimmen von mindestens einem Parameter des an das biologische Gewebe anzulegenden Stimulationsimpulses ausgehend von der Übertragungsfunktion.

6. Verfahren nach Anspruch 5, wobei der Schritt c. die Bestimmung des Stimulationsimpulsparameters umfasst, derart, dass mindestens ein effektiv an das biologische Gewebe angelegter Impuls einen Wert annimmt, der gleich einem Bezugswert mit einer vordefinierten Fehlermarge ist.

7. Verfahren nach einem der Ansprüche 5 oder 6, wobei die Schritte b. und c. durch Berechnungsmittel des elektrischen Stimulators durchgeführt werden.

## Claims

1. An electrical stimulator (SSE) comprising at least:

   - an electrode, or electrode array, intended to come into contact with a biological tissue (TB) that is to be stimulated;
   - an electronic control circuit (CP) for generating stimulation pulses that will be applied to the tissue (TB) via the electrode or electrodes;
   - a means (BMI) for generating at least one electrical signal to be applied to a biological tissue (TB) that is to stimulated and for measuring a response of said biological tissue to said or each said electrical signal;
   - a means (BGS) for generating a said stimulation pulse (X, $V_{STIM}$) to be applied to said biological tissue; **characterized in that** it comprises:
   - a calculation means (MC1, MC2) for estimating, based on the knowledge of said or of each said electrical signal and on the corresponding response of said biological tissue, at least one parameter of an electrical model of said biological tissue and its interface with said electrode or electrodes and for determining, using said model, at least one parameter of a stimulation pulse to be applied to said biological tissue by means of said electrical stimulator, and designed to estimate a transfer function (H) of said electrical model, said transfer function linking the voltage (VTISSU) or the current applied to the tissue to the stimulation voltage (VSTIM) or current, and to determine at least one parameter of said stimulation pulse to be applied to the biological tissue on the basis of said transfer function and of a predetermined reference pulse (Y, V'$_{TISSU}$).

2. Electrical stimulator as claimed in one of the preceding claims, in which said calculation means is designed to determine said stimulation pulse parameter in such a way that at least one parameter of the pulse actually applied to the biological tissue (Y, $V_{TISSU}$) adopts a value equal to a reference value, with a predefined margin of error.

3. Electrical stimulator as claimed in one of the preceding claims, of implantable type.

4. Electrical stimulator as claimed in claim 3, chosen from:

   - an implantable apparatus adapted for deep brain stimulation;
   - an implantable apparatus adapted for retinal stimulation;
   - an implantable apparatus adapted for stimulating the vagus nerve;

      - an implantable apparatus adapted for cortical stimulation; and
      - an implantable apparatus adapted for muscle stimulation.

5. A method for calibrating an electrical stimulator of a biological tissue (TB), as claimed in one of claims 1 to 4 comprising the steps involving:

   a. using said electrical stimulator (SSE) for applying at least one electrical signal to said biological tissue and for measuring the response of said biological tissue to said or to each said electrical signal;
   b. from knowledge of said or of each said electrical signal and of the corresponding response of said biological tissue, automatically estimating at least one parameter of an electrical model of said biological tissue and of the electrode or electrodes; and
   c. using said electrical model to determine automatically at least one parameter of a stimulation pulse ($V_{STIM}$) to be applied to said biological tissue using said electrical stimulator and estimate a transfer function of the biological tissue, said transfer function linking the voltage (VTISSU) or the current applied to the tissue to the stimulation voltage (VSTIM) or current and of its interface with said electrode or electrodes of the electrical stimulator, and to determine at least one parameter of said stimulation pulse to be applied to said biological tissue on the basis of said transfer function.

6. The method as claimed in claim 5, in which step c involves determining said parameter of the stimulation pulse in such a way that at least one parameter of the pulse actually applied to the biological tissue adopts a value equal to a reference value, with a predefined margin of error.

7. The method as claimed in one of claims 5 or 6, in which said steps b and c are implemented by calculation means of said electrical stimulator.

Fig. 1

Fig. 2

Fig. 3A

Fig. 3B

Fig. 4A

Fig. 4B

Fig. 5

Fig. 6A

Fig. 6B

Fig. 9A

Fig. 9B

Fig. 7

$X(j\omega)$ — $\longrightarrow$ $\boxed{H(j\omega)}$ $\longrightarrow$ $Y(j\omega)$

*Fig. 8*

$Y'(t)$ $\longrightarrow$ $\bigcirc$ $\xrightarrow{\varepsilon(t)}$ $\boxed{PID}$ $\longrightarrow$ $X(t)$

$Y(t)$ $\boxed{H(j\omega)}$

*Fig. 10*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Injury and Excitation by Electric Currents - A. The Balanced Pulse-Pair Waveform. **JOHN C LILLY.** Electrical Stimulation of the Brain. Univ. of Texas Press for Hogg Foundation for Mental Health, 1961, 60-64

- **IONESCU C et al.** Activating Paralyzed Muscles using Adaptive Control and a Neuroprosthetic Technique. *IEEE International Conference on Automation, Quality and Testing, Robotics,* 2006
- **M. MIN et al.** Broadband excitation for short-time impedance spectroscope. *Physiol. Meas.,* 2008, vol. 29, S185-S192